(19) Europäisches Patentamt
European Patent Office
Office européen des brevets

(11) Veröffentlichungsnummer: **0 265 824 A2**

## (12) EUROPÄISCHE PATENTANMELDUNG

(21) Anmeldenummer: **87115399.5**

(22) Anmeldetag: **21.10.87**

(51) Int. Cl.4: **C07F 9/09**, A61K 7/075, C11D 1/88

(30) Priorität: **29.10.86 DE 3636750**

(43) Veröffentlichungstag der Anmeldung: **04.05.88 Patentblatt 88/18**

(84) Benannte Vertragsstaaten: **AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Möller, Hinrich, Dr.**
**Schumannstrasse 11**
**D-4019 Monheim(DE)**
Erfinder: **Zeidler, Ulrich, Dr.**
**Heinrich-Lersch-Strasse 19**
**D-4000 Düsseldorf 13(DE)**

### (54) Amphotere und zwitterionische Phosphattenside.

(57) Amphotere und zwitterionische Phosphattenside der Formel (I)

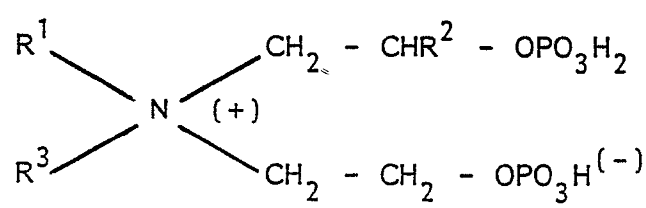

(I)

in welchen entweder $R^1$ oder $R^2$ eine langkettige Alkylgruppe mit 6 bis 22 C-Atomen ist oder $R^1$ eine Gruppe $R^1$-$(OC_nH_{2n})_x$-ist, können hergestellt werden

a) aus tertiären Bis-(2-hydroxyalkyl)-aminen der Formel (II)

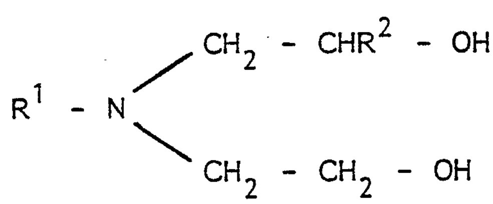

(II)

durch Phosphatierung und Alkylierung zur Einführung der Gruppe $R^3$ oder

b) aus tertiären Hydroxyalkylaminen der Formel (III)

EP 0 265 824 A2

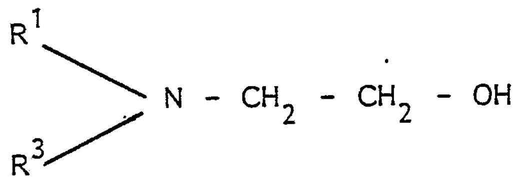 (III)

durch Phosphatierung, Umsetzung mit einem Epoxid der Formel

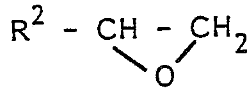

und erneute Phosphatierung. Die Produkte eignen sich als hautverträgliche waschaktive Komponenten in Körperreinigungs-und Haarwaschmitteln.

## "Amphotere und zwitterionische Phosphattenside"

Die Erfindung betrifft neue amphotere, zwitterionische, Phosphatgruppen enthaltende oberflächenaktive Verbindungen, die eine tertiäre Aminogruppe oder quartäre Ammoniumgruppe sowie zwei oder drei Phosphatestergruppen im Molekül enthalten sowie ein Verfahren zur Herstellung dieser Tenside.

Phosphobetaine mit einer quartären Ammoniumgruppe und einer Phosphatestergruppe sind mehrfach beschrieben. Aus US-PS 4,215,064 sind z. B. Verbindungen bekannt, die aus reaktiven Chloralkyl-und Epoxyalkyl-phosphorsäureestern und tertiären Aminen zugänglich sind. Aus Chem. Abstr.98:125401m sind Phosphobetaine bekannt, die durch Reaktion von 3-Chlor-2-hydroxypropyl-alkyl($C_{18}$)-dimethylammoniumchlorid mit Dinatriumhydrogenphosphat erhalten werden.

Amphotere und zwitterionische Tenside mit zwei oder drei Phosphatestergruppen im Molekül sind bisher nicht bekannt. Es wurde nun ein neues, einfaches Verfahren zur Herstellung amphoterer und zwitterionischer Tenside gefunden, die eine tertiäre Aminogruppe oder eine quartäre Ammoniumgruppe sowie zwei oder drei Phosphatestergruppen im Molekül enthalten. Diese neuen Phosphattenside zeichnen sich durch wertvolle Anwendungseigenschaften aus.

Gegenstand der Erfindung sind amphotere und zwitterionische Phosphat-Tenside der Formel

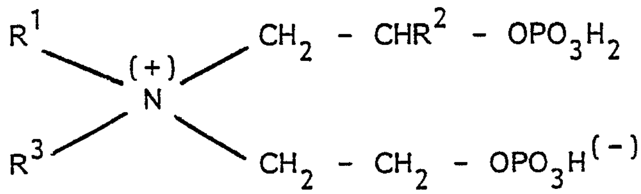

(I)

a) in der $R^1$ eine Alkylgruppe mit 6 bis 22 C-Atomen eine Gruppe $R^1(OC_nH_{2n})_x$-, worin n = 2 bis 4 und x = 1 bis 6 ist oder eine Gruppe $R^4$ -CONH$(CH_2)_m$-, worin $R^4$ eine Alkylgruppe mit 5 bis 21 C-Atomen und m = 2 bis 4 ist, $R^2$ Wasserstoff und $R^3$ Wasserstoff, eine Alkylgruppe mit 1 bis 4 C-Atomen oder eine Gruppe -$(C_nH_{2n}O)_x$-H oder $(C_nH_{2n}O)_x$-$PO_3H_2$, worin n = 2 bis 4 und x = 1 bis 6 ist, darstellt oder

b) in der $R^1$ eine Alkylgruppe mit 1 bis 4 C-Atomen oder eine 2-Hydroxyalkylgruppe mit 2 bis 4 C-Atomen, $R^2$ eine Alkylgruppe mit 6 bis 22 C-Atomen und $R^3$ die vorgenannte Bedeutung hat und deren Gemische mit entsprechend substituierten Phosphat-Tensiden der Formel (I), in welchen die Gruppe -$PO_3H_2$ durch Wasserstoff ersetzt ist sowie deren neutrale und basische Salze.

Wenn $R^3$ Wasserstoff ist, handelt es sich um amphotere Tenside, die unterhalb eines isoelektrischen pH-Wertes überwiegend kationische und oberhalb dieses isoelektrischen Punktes vorwiegend anionische Eigenschaften aufweisen. Wenn $R^3$ ein Substituent ist, handelt es sich um sogenannte zwitterionische Tenside, die eine kationische quartäre Ammoniumgruppe und anionische Phosphatgruppen enthalten.

Die erfindungsgemäßen Tenside der Formel (I) sind oberflächenaktiv, da jeweils eine der Gruppen $R^1$, oder $R^2$ eine Alkylgruppe mit 6 bis 22 C-Atomen ist. Sie zeichnen sich durch ein gutes Schäumvermögen, eine gute Verträglichkeit mit anionischen, kationischen und nichtionischen Tensiden sowie auch mit wasserlöslichen Polymeren verschiedener Ionogenität aus. Ihre wäßrigen Lösungen lassen sich durch Zusätze von Fettsäurealkanolamiden, z. B. von $C_{12}$-$C_{18}$-Fettsäure-mono-und diethanolamiden und einfachen Salzen wie NaCl oder $Na_2SO_4$ verdicken. Darüber hinaus weisen sie eine sehr gute Haut-und Schleimhautverträglichkeit auf und eignen sich daher in besonderer Weise als waschaktive Komponente in Körperreinigungs-und Haarwaschmitteln.

Bevorzugte anwendungstechnische Eigenschaften weisen solche erfindungemäßen zwitterionischen Tenside der Formel (I) auf, in welchen $R^1$ und $R^3$ eine Methylgruppe, $R^2$ eine Alkylgruppe mit 8 bis 16 C-Atomen ist und deren Gemische mit entsprechend substituierten Tensiden der Formel (I), bei welchen die Gruppe -$PO_3H_2$ durch Wasserstoff ersetzt ist sowie deren neutrale und basische Salze.

Die Herstellung der erfindungsgemäßen amphoteren und zwitterionischen Diphosphat-Tenside erfolgt nach einem einfachen Verfahren. Dieses zeichnet sich dadurch aus, daß man tertiäre Bis-(2-hydroxyalkyl)-amine der Formel (II)

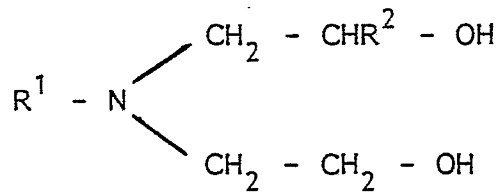

(II)

in der $R^1$ und $R^2$ die für Formel (I) angegebene Bedeutung haben, mit Polyphosphorsäure, Phosphoroxytrichlorid oder Phosphorpentoxid phosphatiert und gegebenenfalls mit Alkylierungsmitteln der Formel $R^3Cl$, $R^3Br$, $R^3_2$ $SO_4$, worin $R^3$ eine Alkylgruppe mit 1 bis 4 C-Atomen ist oder mit x Mol eines Alkylenoxids der Formel $C_2H_{2n}O$ unter Bedingungen umsetzt, die zur Quaternierung des tertiären Amins führen.

Geeignete Bis-(2-hydroxyalkyl)-amine der Formel (II) sind z. B. die Anlagerungsprodukte von 2 Mol Ethylenoxid an Fettamine mit 6 bis 22 C-Atomen, die Kondensationsprodukte von Fettsäuren mit 6 bis 22 C-Atomen mit 1 Mol N,N-Bis-(2-hydroxyethyl)-alkylen-($C_2$-$C_4$)-diaminen und die Kondensationsprodukte von 2-Hydroxyethyl-alkyl($C_1$-$C_4$)-aminen mit Alpha-Epoxyalkanen mit 8 bis 24 C-Atomen.

Die Phosphatierung und die gegebenenfalls durchzuführende Quaternierung können in beliebiger Reihenfolge durchgeführt werden. Man kann also auch zuerst das Bis-(2-hydroxyalkyl)-amin der Formel (II) durch Umsetzung mit einem Alkylierungsmittel oder mit einem $C_2$-$C_4$-Alkylenoxid in eine quartäre Ammoniumverbindung der Formel (IV)

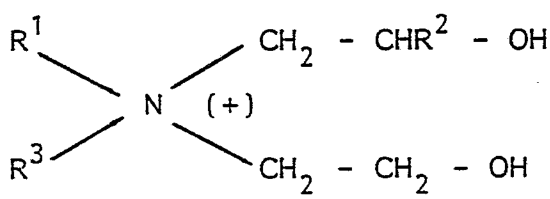

(IV)

überführen und diese mit Polyphosphorsäure, Phosphoroxytrichlorid oder Phosphorpentoxid im Überschuß phosphatieren.

Die Quaternierung mit den genannten Alkylierungsmitteln oder mit einem Alkylenoxid kann in wäßriger oder wäßrig-alkoholischer Lösung nach den üblichen, dafür bekannten Verfahren durchgeführt werden. Bei der Alkylierung mit $R^3Cl$, $R^3Br$ oder $R^3_2$ $SO_4$ werden die freien tertiären Amine eingesetzt, die Quaternierung mit Alkylenoxiden wird vorzugsweise in neutraler oder schwach saurer, wäßriger oder wäßrig-alkoholischer Lösung durchgeführt.

Die Phosphatierung der Bis-(2-hydroxyalkyl)-amine der Formel (II) erfolgt nach den dafür bekannten und üblichen Verfahren durch Umsetzung mit Polyphosphorsäure, Diphosphorsäure, Phosphorpentoxid oder Phosphoroxychlorid.

Bevorzugt wird Polyphosphorsäure mit einem hohen $P_2O_5$-Gehalt verwendet. Diese wird bevorzugt im Überschuß eingesetzt, ein Molverhältnis von 1,5 bis 3, bevorzugt 2 Mol $P_2O_5$ zu 1 Mol des Bis-(2-hydroxyalkyl)-amins hat sich als sehr geeignet erwiesen. Dabei wird ohne Lösungsmittel bei Temperaturen von 40 bis 150 °C gearbeitet. Die Phosphatierung mit $POCl_3$ wird bevorzugt bei Temperaturen von 0 bis 50 °C, vorzugsweise in einem Lösungsmittel wie Tetrahydrofuran oder Diethylenglykoldimethylether unter Mitverwendung einer Hilfsbase wie z. B. Pyridin oder Triethanolamin zur Bindung der dabei entstehenden Salzsäure durchgeführt.

Auch mit Orthophosphorsäure kann ohne Lösungsmittel und bei Anwendung von Temperaturen über 150 °C eine Phosphatierung erreicht werden.

Die Phosphatierung der quartären Ammoniumverbindungen der Formel (IV) erfolgt bevorzugt in einem organischen, wassermischbaren, von Hydroxylgruppen freien Lösungsmittel, z. B. in Tetrahydrofuran, Dioxan, 1,2-Dimethoxyethan oder Diethylenglykoldimethylether. Bei dieser Arbeitsweise werden die höchsten Phosphatierungsgrade erreicht.

Eine weitere Möglichkeit zur Herstellung solcher zwitterionischer Phosphattenside der Formel (I), bevorzugt solcher, in welchen $R^1$ eine Alkylgruppe mit 1 bis 4 C-Atomen und $R^2$ eine Alkylgruppe mit 6 bis 22 C-Atomen ist, besteht darin, daß man tertiäre Hydroxyalkylamine der Formel (III)

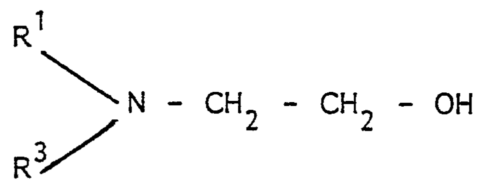

(III)

zunächst mit Polyphosphorsäure, $POCl_3$ oder $P_2O_5$ phosphatiert, dann mit einem langkettigen Epoxid der Formel

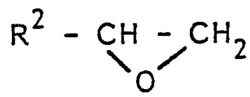

umsetzt und das Umsetzungsprodukt erneut phosphatiert. Die Phosphatierung des tertiären Hydroxyalkylamins der Formel (III) braucht dabei nur mit 0,05 bis 0,2 Mol P des Phosphatierungsreagens pro Mol des Hydroxyalkylamins zu erfolgen, die Phosphatierung des Umsetzungsproduktes mit dem Epoxid wird bevorzugt mit einem Überschuß des Phosphatierungsmittels durchgeführt. Es hat sich z. B. bewährt, insgesamt ca. 2 Mol $P_2O_5$, bevorzugt in Form von Polyphosphorsäure, auf 1 Mol des Umsetzungsproduktes anzuwenden.

Nach dem erfindungsgemäßen Verfahren werden Gemische der erfindungsgemäßen Diphosphat-Tenside der Formel (I) mit entsprechend substituierten Monophosphat-Tensiden der Formel (I), bei welchen die Gruppe $-PO_3H_2$ durch Wasserstoff ersetzt ist, erhalten. Dies ist dadurch bedingt, daß unter den genannten Reaktionsbedingungen keine vollständige Phosphatierung aller Hydroxylgruppen erreicht wird. Wenn man den Phosphatierungsgrad als den Anteil der in eine $-OPO_3H_2$-Gruppe überführten Hydroxylgruppen des Ausgangsproduktes ansieht, so ist bei einem Phosphatierungsgrad oberhalb 0,5 auf jeden Fall die Bildung der erfindungsgemäßen Diphosphat-Tenside der Formel (I) erfolgt.

Der Phosphatierungsgrad läßt sich durch Anwendung eines Überschusses des Phosphatierungsmittels steigern. Aus den analytischen Daten des Phosphatierungsproduktes läßt sich der Phosphatierungsgrad wie folgt abschätzen:

$$\text{Phosphatierungsgrad} = \frac{P(\text{gesamt}) - P(\text{anorg.})}{1/n\ P(\text{gesamt})}$$

Die Werte für P(gesamt) (= Gew.-% Gesamtphosphor) und für P(anorg.) (= Gew.-% anorganisch gebundener Phosphor) lassen sich nach bekannten analytischen Methoden bestimmen. n ist die Molmenge Phosphor, die pro Hydroxylgruppe eingesetzt wurde. Die Aufarbeitung der Reaktionsgemische nach der Phosphatierung erfolgt durch Zugabe von Wasser und Erhitzen auf 80 bis 100 °C bis die Polyphosphat-und Diphosphat-Strukturen hydrolysiert sind. Diese Reaktion ist nach 30 bis 60 Minuten beendet. Dann wird so viel Alkalihydroxid oder Alkalicarbonat zugesetzt, wie für die Bildung des gewünschten Salzes erforderlich ist. Zur Herstellung der entsprechenden Kaliumsalze wird Kaliumhydroxidlösung, zur Herstellung von Ammoniumsalzen wäßrige Ammoniaklösung zugesetzt. Es können auch Alkanolamin-Salze, z. B. durch Zugabe von Monoethanolamin, Diethanolamin, Triethanolamin, Isopropanolamin oder andere Aminsalze z. B. mit Morpholin, Piperidin und anderen Aminen hergestellt werden.

Durch Zugabe von kleineren Mengen Calciumhydroxid kann man auch anorganisches Orthophosphat in Form des schwerlöslichen Ca-Salzes ausfällen und auf diese Weise aus dem Produkt entfernen.

Die folgenden Beispiele sollen den Gegenstand der Erfindung näher erläutern, ohne ihn hierauf zu beschränken.

**Beispiele**

1. Phosphatierung von 2-Hydroxyethyl-2-hydroxyhexadecylmethylamin

39,4 g (0,125 Mol) 2-Hydroxyethyl-2-hydroxyhexadecylmethylamin (aus 2-Methylaminoethanol und Alpha-Hexadecenoxid) mit einer Aminzahl von 184 wurden bei 20 °C mit 42,2 g (0,25 Mol $P_2O_5$) Polyphosphorsäure (84 Gew.-% $P_2O_5$) verrührt und innerhalb von 15 Minuten auf 150 °C erwärmt. Nach Abkühlung auf 80 °C wurde das Reaktionsgemisch in ca. 350 g Wasser aufgenommen und mit 50 Gew.-%iger wäßriger Natronlauge neutralisiert. Ungelöste Nebenprodukte wurden durch Filtration abgetrennt. Der Phosphatierungsgrad betrug 0,53.

2. 2-Hydroxyethyl-2-hydroxyhexadecyl-dimethylammoniumphosphat

200 g der Lösung nach Beispiel 1 wurden mit 20 g Methylchlorid im Autoklaven bei 100 °C und 5 bar $N_2$-Druck umgesetzt. Nach 3,5 Stunden war die Reaktion beendet. Die erhaltene Lösung wurde unter vermindertem Druck zur Trockene eingeengt, dabei wurde eine farblose, wachsartige Substanz erhalten. Diese zeigte folgende Analysendaten:

$H_2O$: 3,2 Gew.-%
$Cl^{(-)}$: 9,1 Gew.-%
Phosphatierungsgrad: 0,53

3. Phosphatierung von Dimethyl-2-hydroxyethyl-2-hydroxyhexadecylammoniumchlorid

Zu einer Mischung aus 33,8 g (0,2 Mol $P_2O_5$) Polyphosphorsäure (84 Gew.-% $P_2O_5$) und 200 ml Diethylenglykoldimethylether wurden unter Rühren bei 80 °C 36,6 g (0,1 Mol) Dimethyl-2-hydroxy-ethyl-2-hydroxyhexadecylammoniumchlorid gegeben. Das Reaktionsgemisch wurde 5 Stunden unter Rühren auf 100 °C erwärmt. Dabei wurde die Mischung homogen. Nach Abdestillieren des Lösungsmittels unter vermindertem Druck wurde der Rückstand mit 400 ml Wasser versetzt, zum Sieden erhitzt und nach dem Abkühlen auf 50 °C mit 50%iger wäßriger Natronlauge auf pH = 7 gestellt und mit Aktivkohle heiß filtriert. Die Lösung zeigte folgende Analysendaten:

P(gesamt): 14,9 Gew.-%
P(anorg.): 8,4 Gew.-%

$$\text{Phosphatierungsgrad} = \frac{\text{P(gesamt)} - \text{P(anorg.)} \times 2}{\text{P(gesamt)}} = 0{,}87$$

4. Fettacyl($C_{12/14}$)-aminopropyl-di-(2-hydroxyethyl)-methylammonium-phosphat

4.1 Vorstufe

Zuerst wurde aus Fettsäure-$C_{12/14}$ (70 : 30) und N,N-bis-(2-hydroxyethyl)-propylen(1,3)-diamin in Toluol unter azeotroper Entfernung des Kondensationswassers das N,N-bis-(2-hydroxyethyl)-N′-Fettacyl($C_{12/14}$)-propylen(1,3)-diamin hergestellt.

4.2 Phosphatierung

88 g (0,25 Mol) des Aminoamids nach 4.1 wurden bei 90 bis 100 °C zu 84,4 g (0,5 Mol $P_2O_5$) Polyphosphorsäure (84 Gew.-% $P_2O_5$) unter Wasserausschluß und Rühren gegeben. Die Mischung wurde innerhalb von 15 Minuten auf 150 °C erwärmt und 30 Minuten bei dieser Temperatur gerührt. Nach dem Abkühlen auf 80 °C wurde das Reaktionsgemisch in 300 g Wasser eingerührt, mit 50 %iger wäßriger Natronlauge auf pH = 7 gestellt und von wenig ungelöstem Nebenprodukt durch Filtration abgetrennt.

4.3 Quaternierung

200 g der Lösung gemäß 4.2 (0,093 Mol) wurden mit 20 g (0,4 Mol) Methylchlorid im Autoklaven bei 100 °C und 5 bar $N_2$-Druck umgesetzt. Die Reaktion war nach 3,5 Stunden beendet. Die stark schäumende Lösung wurde unter vermindertem Druck zur Trockene eingeengt, dabei wurde eine farblose, wachsartige Substanz erhalten. Diese zeigte folgende Analysendaten:

P(gesamt): 13 Gew.-%
P(anorg.): 8,9 Gew.-%
$H_2O$: 5,5 Gew.-%
$Cl^-$: 7,1 Gew.-%

$$\text{Phosphatierungsgrad} = \frac{\text{P(gesamt)} - \text{P(anorg.)} \times 2}{\text{P(gesamt)}} = 0{,}63$$

5. Phosphatiertes Dimethyl-2-hydroxyethyl-2-hydroxyhexadecyl-ammoniumphosphat

20 g (0,118 Mol $P_2O_5$) Polyphosphorsäure (84 Gew.-% $P_2O_5$) wurden unter Rühren mit 44,6 g (0,5 Mol) 2-Dimethylaminoethanol versetzt und nach Abklingen der exothermen Reaktion 2 Stunden zum Sieden erhitzt. Nach Abkühlen auf 80 °C wurden 120 g (0,5 Mol) 1,2-Epoxyhexadecan eingerührt und die Temperatur langsam auf 100 °C gesteigert. Am Ende der auftretenden exothermen Reaktion wurde noch 1 Stunde bei 100 °C und 0,5 Stunden bei 135 °C gerührt, danach mit 100 ml Diethylenglykoldimethylether versetzt und eine weitere Stunde zum Sieden erhitzt. Die auf 100 °C abgekühlte gelbbraune Lösung wurde in 149 g (0,88 Mol $P_2O_5$) Polyphosphorsäure, die vorher auf 80 °C erwärmt worden war, eingetragen und die Mischung 2 Stunden bei 130 °C gerührt. Anschließend wurde das Lösungsmittel unter vermindertem Druck abdestilliert, der Rückstand in 500 ml Wasser aufgenommen, die Lösung 0,5 Stunden zum Sieden erhitzt und nach dem Neutra lisieren mit Natronlauge (50%ig) zur Trockene eingedampft. Es wurde ein grau-weißes, festes Produkt erhalten.

P(gesamt): 16,9 Gew.-%
P(anorg.): 11,2 Gew.-%
Phosphatierungsgrad: 0,68

In Analogie zu den Beispielen 1 bis 5 wurden noch folgende Substanzen hergestellt:

6. Phosphatiertes Dimethyl-2-hydroxyethyl-2-hydroxytetradecylammoniumchlorid (analog Beispiel 3)

Phosphatierungsgrad: 0,89

7. Phosphatiertes Bis-2-hydroxyethyl-3-($C_{12/14}$-fettsäureamido)-propyl-methyl-ammoniumchlorid (analog Beispiel 3)

Phosphatierungsgrad: 0,98

8. Phosphatiertes $C_{12/14}$-Alkyl-poly(3,6)-oxyethyl-bis-(2-hydroxyethyl)-methyl-ammoniumchlorid

Ausgehend von einem $C_{12/14}$-Alkylpoly(3,6)glykolethersulfat, Na-Salz wurde durch Alkylierung von Diethanolamin das $C_{12/14}$-Alkyl-poly(3,6)-oxyethyl-bis-(2-hydroxyethyl)amin hergestellt. Dieses wurde mit Methylchlorid quaterniert und das quartäre Ammoniumsalz analog Beispiel 3 phosphatiert.

9. Phosphatiertes Kokosalkyl-tris-(2-hydroxyethyl)-ammoniumchlorid

Kokosalkyl-bis-(2-hydroxyethyl)-aminhydrochlorid wurde mit einer wäßrig-alkoholischen Lösung (Propanol-2 : $H_2O$ = 1 : 1) bei 85 °C mit Ethylenoxid quaterniert. Das quartäre Ammoniumchlorid wurde analog Beispiel 3 mit Polyphosphorsäure (3 Mol $P_2O_5$ pro Mol) phosphatiert. Phosphatierungsgrad: 0,85

10. Phosphatiertes 2-Dodecyloxyethyl-tris-2-hydroxyethyl-ammoniumchlorid

Ausgehend von 2-Dodecyloxyethylsufat, Na-Salz wurde durch Alkylierung von Diethanolamin das 2-Dodecyloxyethyl-bis-(2-hydroxyethyl)-amin hergestellt, in das Hydrochlorid überführt und mit Ethylenoxid in wäßrig alkoholischer Lösung quaterniert. Die Phosphatierung erfolgte mit Polyphosphorsäure (3 Mol $P_2O_5$ pro Mol).
Phosphatierungsgrad: 0,93

**Ansprüche**

1. Amphotere und zwitterionische Phosphattenside der Formel (I)

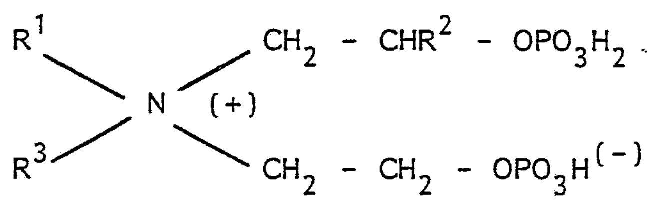

( I )

a) in der $R^1$ eine Alkylgruppe mit 6 bis 22 C-Atomen, eine Gruppe $R^1(OC_nH_{2n})_x$-, worin n = 2 bis 4 und x = 1 bis 6 ist oder eine Gruppe $R^4$ -$CONH(CH_2)_m$-, worin $R^4$ eine Alkylgruppe mit 5 bis 21 C-Atomen und m = 2 bis 4 ist, $R^2$ Wasserstoff und $R^3$ Wasserstoff, eine Alkylgruppe mit 1 bis 4 C-Atomen oder eine Gruppe -$(C_nH_{2n}O)_x$-H oder $(C_nH_{2n}O)_x$-$PO_3H_2$, worin n = 2 bis 4 und x = 1 bis 6 ist, darstellt oder

b) in der $R^1$ eine Alkylgruppe mit 1 bis 4 C-Atomen oder eine 2-Hydroxyalkylgruppe mit 2 bis 4 C-Atomen, $R^2$ eine Alkylgruppe mit 6 bis 22 C-Atomen und $R^3$ die vorgenannte Bedeutung hat und deren Gemische mit entsprechend substituierten Phosphat-Tensiden der Formel (I), in welchen die Gruppe -$PO_3H_2$ durch Wasserstoff ersetzt ist sowie deren neutrale und basische Salze.

2. Amphotere und zwitterionische Phosphattenside der Formel (I) gemäß Anspruch 1, dadurch gekennzeichnet, daß $R^1$ und $R^3$ eine Methylgruppe und $R^2$ eine Alkylgruppe mit 8 bis 16 C-Atomen ist und deren Gemische mit entsprechend substituierten Phosphattensiden der Formel (I), bei welchen die Gruppe $PO_3H_2$ durch Wasserstoff ersetzt ist sowie deren neutrale und basische Salze.

3. Verfahren zur Herstellung von amphoteren und zwitterionischen Phosphattensiden der Formel (I)

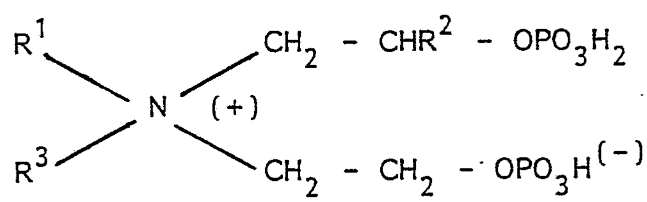

( I )

a) in der $R^1$ eine Alkylgruppe mit 6 bis 22 C-Atomen eine Gruppe $R^1(OC_nH_{2n})_x$-, worin n = 2 bis 4 und x = 1 bis 6 ist oder eine Gruppe $R^4$ -$CONH(CH_2)_m$-, worin $R^4$ eine Alkylgruppe mit 5 bis 21 C-Atomen und m = 2 bis 4 ist, $R^2$ Wasserstoff und $R^3$ Wasserstoff, eine Alkylgruppe mit 1 bis 4 C-Atomen oder eine Gruppe -$(C_nH_{2n}O)_x$-H oder $(C_nH_{2n}O)_x$-$PO_3H_2$, worin n = 2 bis 4 und x = 1 bis 6 ist, darstellt oder

b) in der $R^1$ eine Alkylgruppe mit 1 bis 4 C-Atomen oder eine 2-Hydroxyalkylgruppe mit 2 bis 4 C-Atomen, $R^2$ eine Alkylgruppe mit 6 bis 22 C-Atomen und $R^3$ die vorgenannte Bedeutung hat und deren Gemische mit entsprechend substituierten Phosphat-Tensiden der Formel (I), in welchen die Gruppe -$PO_3H_2$ durch Wasserstoff ersetzt ist, dadurch gekennzeichnet, daß man

a) tertiäre Bis-(2-hydroxyalkyl)-amine der Formel (II)

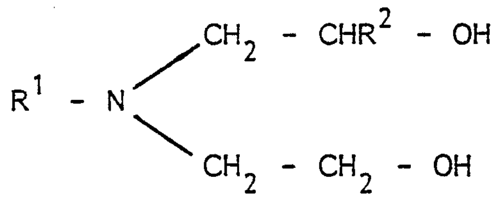

(II)

in beliebiger Reihenfolge mit Polyphosphorsäure, $POCl_3$ oder $P_2O_5$ phosphatiert und gegebenenfalls mit Alkylierungsmitteln der Formel $R^3Cl$, $R^3Br$, $R^3_2SO_4$, worin $R^3$ eine Alkylgruppe mit 1 bis 4 C-Atomen ist oder mit x Mol eines Alkylenoxids der Formel $C_nH_{2n}O$ quaterniert oder daß man

b) tertiäre Hydroxyalkylamine der Formel (III)

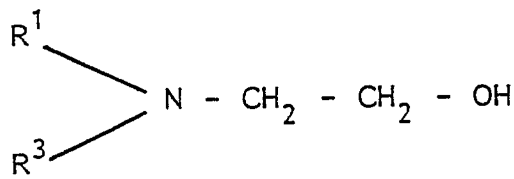

(III)

mit Polyphosphorsäure, $POCl_3$ oder $P_2O_5$ phosphatiert, mit einem Epoxid der Formel

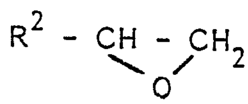

umsetzt und das Umsetzungsprodukt erneut phosphatiert.

4. Verwendung der amphoteren und zwitterionischen Phosphattenside der Formel (I) gemäß der Ansprüche 1 oder 2 als waschaktive Komponenten in Körperreinigungs-und Haarwaschmitteln.